## Europäisches Patentamt
## European Patent Office
## Office européen des brevets

(19)

(11) Publication number: **0 078 990**
**B1**

(12) **EUROPEAN PATENT SPECIFICATION**

(45) Date of publication of patent specification: **04.02.87**

(51) Int. Cl.⁴: **G 01 N 27/30,** G 01 N 27/48,
G 01 N 27/40, G 01 N 33/48

(21) Application number: **82109957.9**

(22) Date of filing: **28.10.82**

(54) **Enzyme electrode membrane wherein enzyme is protectively encapsulated and method of making same.**

(30) Priority: **05.11.81 US 318625**

(43) Date of publication of application:
**18.05.83 Bulletin 83/20**

(45) Publication of the grant of the patent:
**04.02.87 Bulletin 87/06**

(84) Designated Contracting States:
**CH DE FR GB IT LI SE**

(56) References cited:
**CH-A- 569 973**
**DE-B-2 638 193**
**GB-A-1 253 770**
**US-A-4 073 713**

(73) Proprietor: **MILES LABORATORIES, INC.**
**1127 Myrtle Street**
**Elkhart Indiana 46514 (US)**

(72) Inventor: **Oberhardt, Bruce J.**
**405-4, Bercado Circle**
**Mishawaka Indiana 46544 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

EP 0 078 990 B1

Courier Press, Leamington Spa, England.

# Description

The present invention in its broadest aspects relates to a membrane suitable for use with an electrochemical sensor and a method of making said novel membrane. The membranes are used in voltametric cells for electrochemical analysis commonly referred to as polarographic cells and mentioned as such hereinafter and are based on a reaction whereby an enzyme converts a substance which is an unknown to be measured into a material which can be measured by way of an electrical signal from the cells.

A wide variety of assay techniques and sensors are available for the measurement of various materials. Of particular interest to the medical field, is the measurement of small amounts of various substances contained in body fluids, such as bloods, in body tissues, in foodstuffs, and the like. Such substances include glucose, urea, uric acid, triglycerides, phospholipids, creatinine, amino acids, lactic acid, xanthine, chondroitin, etc. The development of a sensor for controlling or monitoring the glucose concentration in blood or other body fluids is particularly important especially for maintaining normal blood glucose levels in a diabetic patient. Typically, blood samples are withdrawn from the patient for an on-line analysis for glucose concentrations using a glucose oxidase electrode with a polarographic detector for the generated hydrogen peroxide. Customarily, such detectors comprise an enzyme electrode for the determination of hydrogen peroxide with an anode, a cathode, an electrolyte, and a membrane of specific composition containing an enzyme that has been immobilized.

Enzymes have been used in conjunction with polarographic cells in instances where the unknown substance to be measured is not electrochemically active itself, but by conversion or reaction of the enzyme with the unknown sample, a reaction product is obtained that may be measured; that is, it is detectable by polarographic means. As stated above, the most common problem of medical interest is the desire to measure glucose in the blood. The problem is that glucose is itself not electrochemically active. However, in the presence of the enzyme glucose oxidase the following reaction takes place:

$$\text{Glucose} + O_2 \xrightarrow{\text{glucose oxidase}} \text{gluconic acid} + \text{hydrogen peroxide } (H_2O_2)$$

The hydrogen peroxide that is generated by this reaction is measurable by a polarographic detector and therefore by appropriate calibration and calculations, it is possible to determine, from the amount of $H_2O_2$ liberated, what the glucose content was in the original specimen or sample. The above discussion is illustrative and applies in general to other enzymes as well.

Generally, a polarographic cell comprises an electrically insulating receptacle, an indicator or sensing electrode electrically contacting a membrane and a reference electrode which is electrically in contact with the membrane. By the expression "contacting" it is intended to include the situation where the contact between membrane and electrode is obtained directly or through a layer of electrolyte. Cells of various designs are widely known and understood in the art. An especially suitable cell for purposes of the invention is shown in Clemens et al, U.S. Patent No. 4,092,233.

In the prior art, in the case of an enzyme membrane structure, it is known to arrange a second hydrophilic membrane at a distance from the first mentioned membrane. In the space between the two membranes, a layer of concentrated enzyme is present. The free face of the second membrane provides the test surface to which the substrate to be tested is applied. This type of enzyme membrane is described in the Annals of the New York Academy of Science, Vol. 102 (1962), pages 29—49. In that article, it was suggested that a pH sensitive electrode could be used to detect gluconic acid produced by the reaction. It was disclosed that the enzyme in such a system could be trapped between two cellulose acetate membranes. Glucose diffuses through the membrane and is converted by the enzyme to gluconic acid when then diffuses both towards the pH sensitive glass and back into the donor solution.

The first mentioned membrane facing the sensing electrode is made up of a material which can be penetrated by the substance to which the sensing electrode is sensitive. For example, this membrane is permeable to the reactants of the enzymatic reaction but impermeable to the enzyme itself. It may be made of cuprophane but in the event that one of the reaction products is a gas at normal pressure and temperature and it is desired to measure via this gas, the membrane may consist of hydrophobic plastic impermeable to ions but slightly permeable to such gases as oxygen, carbon dioxide or ammonia. Numerous plastics having such properties are known including silicone rubber, tetrafluoroethylene and the like.

In a later type of polarographic cell developed by Clark and described in U.S. Patent No. 3,539,455, the enzyme is placed on the electrode side of the membrane, and a platinum anode measures the hydrogen peroxide produced. Glucose, a low molecular weight species, passes through the membrane and reacts with the enzyme, but interfering high molecular weight substances such as catalase and peroxidase do not. It is disclosed that the enzymes may be held in a thin film directly between the platinum surface and the membrane by placing the enzyme on a porous film which has spaces large enough to hold enzyme molecules. However, cellophane membranes will not prevent low molecular weight interfering materials such as uric acid or ascorbic acid from reaching the sensing electrode. Clark suggests a dual electrode system to overcome that problem. The compensating electrode, without an enzyme present, gives a signal for the interfering substances while the enzyme electrode detects both the hydrogen peroxide an the interference. By calculation, the glucose level is determined. Such a dual sensor system, however, may encounter difficulties in the matching of the two cells.

It was then proposed to have an enzyme electrode which employs a thin filger membrane to prevent passage of low molecular weight interfering materials, such as uric acid and ascorbic acid, while permitting hydrogen peroxide to pass therethrough with minimum hindrance. There exist membrane materials, such as silicone rubber and cellulose acetate, which permit passage of hydrogen peroxide but which are effective barriers to interfering substances. Since this type of membrane must be placed between the sensing electrode and some component of the sensing system, it follows that in order to measurement equilibrium to be as rapid as possible, the membrane must be as thin as possible while still retaining its selectivity. In the case of a hydrogen peroxide sensing probe, this membrane will need to be less than 2 micrometres (hereafter called microns) thick. A membrane of this thickness is difficult to use in practice because of its insufficient strength.

The art then turned to depositing the material in a thin layer on a porous substructure to provide the necessary strength while at the same time being of little hindrance to hydrogen peroxide passage, and the weak interference rejecting layer can be thin to enhance speed of response.

As described in Newman, U.S. Patent No. 3,979,274, in a laminated two-ply membrane, an enzyme adhesive is used to bond the two-plies together. The membrane includes a support layer which controls substrate diffusion and serves as a barrier to high molecular weight substances, an enzyme preparation for reacting with the unknown and for bonding the layers together, and an essentially homogeneous layer that serves as a barrier to interfering low molecular weight materials but permits hydrogen peroxide to pass through. However in this development, it is necessary to make a sandwich consisting of two membranes with a layer of enzyme between, the enzyme acting as the adhesive or bonding agent. In this type of arrangement, the use of too much enzyme could slow down the diffusion of the diffusing species to an unacceptable amount. If a thinner layer of enzyme is used, there is acceptable diffusion, but the loading of enzyme may not be adequate.

A still later development came in British Patent No. 1,442,303 (Radiometer) wherein there was proposed a composite membrane which is an inhomogeneous membrane formed as a unit. The membrane has two different strata, one has a thickness of less than 5 microns and the other is sufficiently thick to provide strength. The enzyme is bonded to a surface of the membrane.

Other prior art has shown a number of disadvantages.

Thus, the method of Koyama et al, *Analytica Chemica Acta*, Vol. 116, pages 307—311 (1980), immobilizes glucose oxidase to a cellulose acetate membrane. This method is more time consuming; it involves more steps and suffers from the disadvantages that a monolayer of molecules would be the maximum possible enzyme load achievable.

The invention described in the present application, however, allows much greater amounts of enzyme to be spacially distributed within the membranes such that much more enzyme is available for reaction with the substrate along the diffusion path of said substrate.

Wingard et al, *The Journal of Biomedical Materials Research*, Vol. 13, pages 921—935 (1979) discloses a platinum screen or wire for immobilization of the enzyme. This would allow greater surface area to be utilized for binding than the method of Koyama et al and hence could employ greater numbers of enzyme molecules. However, the approach of Wingard is also limited to a monolayer of enzyme and capable of sustaining high conversion rates of substrate diffusing through the open spaces in the platinum screen near the surface of the platinum wire only. Hence, this prior art cannot achieve the theoretical conversion rates possible with an enzyme spacially distributed throughout a membrane through which the substrate diffuses, as is obtainable by following this invention.

In accordance with the present invention, the need to prepare a discrete enzyme layer is eliminated by incorporating the enzyme directly into the membrane in a manner whereby the enzyme is homogeneously dispersed throughout and protectively encapsulated in the membrane and immobilized therein.

A number of advantages characterize the present invention including protection of a solvent sensitive enzyme and therefore, the capability of using enzymes which currently have limited utility. Also the new membrane of the invention readily lends itself to being applied to a surface by simple dipping, painting or spraying whereby the desired enzyme containing layer may be obtained.

The present invention also provides a spatially distributed population of enzyme molecules within a membrane structure so as to achieve high rates of conversion of substrate molecules without substantially interfering with their diffusion within the membrane.

In addition, a greater uniformity of enzyme concentration may be achieved by the homogeneous distribution in a membrane than by sandwiching bulk enzyme between two layers.

The principles involved in the present invention may be more fully understood with reference to the analysis of blood for glucose content. The liquid portion of blood consists of proteins, lipids, and other substances. Non-electrolytes are present such as glucose, enzymes such as catalase, electrolytes such as ascorbic acid (vitamin C) and various metallic salts made up of cations of sodium, potassium, magnesium, calcium, iron and copper, and anions such as chlorides, phosphates, bicarbonates, and carbonates. The phosphates, carbonates and bicarbonates operate as buffers to maintain the pH of blood at a fixed level under normal conditions. If a sample of blood were placed on one side of a membrane in a cell and an aqueous solution of the enzyme glucose oxidase and oxygen on the other side of the membrane, certain low molecular weight materials will pass from the blood through the membrane to the glucose oxidase solution. The high molecular weight materials such as the enzymes will not pass through the membrane. The rates of permeability of the various materials through the membrane are fixed because of the nature of the membrane. In this invention, the relatively thin phase has a molecular cut off of approximately 300. This means that materials of a molecular weight of greater than about 300 will not pass through.

Glucose, a low molecular weight material, will pass through the membrane and react with the enzyme glucose oxidase in the presence of oxygen to form gluconolactone and hydrogen peroxide. Gluconolactone in the presence of water will hydrolyze spontaneously to form gluconic acid.

Gluconic acid and hydrogen peroxide being relatively low molecular weight materials compared to the enzyme glucose oxidase will pass through the membrane. Catalase and peroxidase which are large enzyme molecules capable of rapidly destroying $H_2O_2$ and which are present in biochemical fluids are prevented from passing through the membrane.

According to the present invention, the membrane may be utilized in a cell for electrochemical analysis comprising, in general, an electrically insulating receptacle, an anode and a cathode as is shown in U.S. Patent No. 4,092,223. The membrane of this invention may also be used in older type devices utilizing a sensing electrode (anode), a reference electrode (cathode) in a space in the receptacle which is separated from the sensing electrode and adopted to hold an electrolyte. Clark, U.S. 3,539,455 is an example of this type. The membrane electrically contacts the electrodes; a path for an electrical current extends between anode and cathode or between the reference electrode and the sensing electrode and the membrane.

The invention provides a generalized method for electrochemical sensing of an analyte utilizing an enzyme for interaction with the analyte to produce an electroactive species. A feature is the provision for incorporation of an enzyme in a membrane such that the enzyme is spatially distributed throughout one or more layers in the membrane in "pockets" so as to protect the enzyme from solvents. This distributed enzyme contained within the membrane structure itself allows more uniform distribution of enzyme to be achieved, is very easy to prepare, readily lends itself to a dip casting process whereby the membrane may be attached directly to a surface, and allows greater amounts of enzyme to be brought into play in the electrochemical conversion of the analyte. A further feature is the ability for utilizing a wide variety of enzymes, even those which are easily damaged by solvents.

The principal problem associated with preparation of distributed enzyme layers is that the membrane material which contains the enzyme typically must be dissolved in a solvent that may damage or destroy the enzyme. The invention described herein overcomes this problem by providing for the inclusion of an enzyme within zones or pockets containing hydrophilic solvent such as for example, buffered water. These zones or pockets are distributed throughout a solution of membrane material in a hydrophobic solvent. As used herein, the terms "hydrophobic" and "hydrophilic" solvents refer to essentially nonpolar and polar solvents, respectively. This allows the enzyme to be distributed throughout the mixture, and yet be protected or encapsulated by its immediate environment which is nonhostile.

The hydrophilic solvent containing enzyme may be finely dispersed in the hydrophobic solvent containing membrane materials by the use of agitation, ultrasonic mixing, or other means. This will allow a homogeneous distribution of "pockets" containing enzyme to be achieved prior to casting. The hydrophobic solvent, by its very nature will not enter the aqueous zones containing enzymes, other than perhaps in trace amounts. These zones will, therefore, protect the enzyme against solvent and therefore insure stability and useful life for the enzyme.

In carrying out the invention, the membrane may be made by casting or spreading the mixture of membrane forming material and of enzyme in hydrophilic "pocket" may typically consist of a solution capable of forming a gel after the membrane has been cast. The use of coupling agents or fixatives (e.g., glutaraldehyde) to attach enzymes to each other, or to proteins contained within the gel, or to the gel material itself, may also be practiced.

The use of additional layers of membrane forming material may be helpful. For example, a denser (smaller pore) layer may be cast on top of the distributed enzyme layer or vice versa. This second layer may be necessary to provide diffusional control of analytes and/or to eliminate interfering species. It is also possible to use additional layers for protective purposes such as to incorporate nonthrombogenic characteristics to the surface for prolonged use in blood.

A membrane exhibiting the properties called for by the present invention can be made of cellulose acetate as well as other materials such as copolymers of cellulose acetate.

It has been determined that a reasonably short measuring time requires that the thickness of the membrane should not exceed, preferably, about 70 microns overall although this can vary depending on the kind of measurement to be carried out. It would be possible to achieve an acceptable short response time for an equilibrium of diffusion, for example, of hydrogen peroxide by designing the membrane to be made up of more than one layer, for example, one thinner, more dense layer of 2 microns and a thicker, less dense layer of 65 microns. If formed of multiple layers, the membrane of the invention will be formed by combining a phase which when cast alone is relatively thick, and which in the composite membrane with a second phase which when cast alone is thinner and relatively nonporous and which in the composite membrane faces the sample; e.g., the blood specimen. There is uniformly distributed throughout the highly porous phase, the pockets of enzyme. In the composite membrane, this enzyme by be distributed throughout both phases. The individual properties of these phases, if cast separately should be as follows: the relatively nonporous phase should have a molecular weight cut off of approximately 300; the highly porous phase should freely pass the substrate of the enzyme (at the surface adjacent to the surface onto which it has been cast) and yet exclude macromolecules such as large proteins.

In order to achieve these properties, the membrane of the invention may be formed in the following manner: A solution of cellulose acetate in a hydrophobic solvent such as methylene chloride is prepared by dissolving powdered cellulose acetate and utilizing continuous stirring until all of the solute is dissolved. Typically, 6% cellulose acetate is utilized, although as little as 3% may be utilized. To this solution is added a second solution. The second solution consists of a gel forming substance, a buffer, and an enzyme. In a typical preparation, 5 parts of the cellulose acetate solution in methylene chloride are added to 1 part of the second solution. The second solution typically consists of 1 milliliter of enzyme solution in an appropriate buffer (e.g., liquid glucose oxidase enzyme) plus 1 to 10 parts of a 1 to 5% gelatin solution in a buffer. A variety of buffers may be utilized. Phosphate buffered saline, for example, may be employed.

The gelatin is kept at an elevated temperature so that gelation does not occur until after the membrane is cast and the gel allowed to cool. If another gel former (e.g., acrylamide) is utilized, activation of the gel forming process should be delayed until after the membrane is cast. Glutaraldehyde or other fixatives may be utilized, by addition directly to the hydrophilic phase. Various gel formers may be used for purposes of the invention including: agarose, gelatin and polyacrylamide.

The mixture of cellulose acetate solution in hydrophobic solvent combined with the aqueous solution of gel former and enzyme, etc., is spun on a vortex type mixer to achieve a homogeneous blending. Alternatively, a mechanical tissue homogenizer or ultrasonic disrupter type homogenizer may be utilized. In some instances, it may be necessary to add some ethanol or methanol prior to blending to achieve a better casting of the membrane. This will depend on flow and viscosity properties and can be varied as will be apparent to the skilled artisan. In most cases, as much as 5% ethanol or methanol (but typically less) was employed.

To provide a truly universal approach which overcomes the possible limitations of ethanol or methanol, another technique is to utilize, in addition to the hydrophobic solvent for cellulose acetate, a second hydrophobic solvent in which cellulose acetate is insoluble such that neither solvent will dissolve in the hydrophilic phase containing enzyme. The second solvent should be less volatile than the first solvent for cellulose acetate (e.g., methylene chloride) so as to persist after partial or complete evaporation of the first solvent thereby assisting in membrane pore formation by phase inversion processes.

In all of the above techniques, it is best to match the specific gravity of solvents as closely as possible. This allows more homogeneous mixtures to be obtained prior to casting.

The homogenized mixture of enzyme distributed in hydrophilic pockets within a hydrophobic membrane mixture is then spread on a surface and allowed to dry. If the enzyme containing solution is prepared with gelatin or agarose, or other gel former, the drying process may also constitute a cooling step necessary to allow the gelling of the enzyme containing micropockets.

A second layer may be employed consisting of membrane forming material (e.g., cellulose acetate) dissolved in hydrophobic solvent (e.g., methylene chloride) or possibly a polar solvent, in some cases, to further control the diffusion of entering analyte species and/or the exclusion of interfering species during assay measurements. The second layer will typically consist of 3% cellulose acetate by weight dissolved in methylene chloride or acetone and may be spread on top of the distributed and protected enzyme layer. This layer may also be applied by a dipping, painting, or spraying process after the first layer has been formed.

A third layer containing highly porous and durable material may also be utilized with or without the second layer to achieve extra mechanical stability. In addition, nonthrombogenic characteristics may be imparted to this third layer of virtue of:

(a) the material itself (as in the case with some types of polyurethanes), or

(b) by inclusion of heparin binding species (e.g., graphite), or

(c) by covalent bonding to the surface prostetic groups capable of preventing thrombus formation (acid polysaccharides such as heparin).

The latter method may be utilized with the second layer, as well, if a third layer is not employed.

When casting the cellulose acetate membrane, any suitable surface which does not interact with or bond to the membrane may be used. Representative surfaces to provide a support for the cast film are glass and some plastics such as polyethylene. The film is cast with conventional equipment whereby it is possible to control the thickness of the resulting film. After being spread on the surface, the cast film is dried. Drying may be accelerated and/or controlled by the use of temperature/humidity chambers.

When utilizing a multiple layer membrane, the thicker layer cellulose acetate membrane containing the enzyme may be cast directly on top of the ultra thin membrane. Since both casting solutions are preferably of the same polymer base, and preferably use the same solvent, there is a diffusion zone of the two phases at the interface or boundary and no clear distinction can be made between the two layers. Since an intermingling or diffusion of the layers is believed to occur, the terms layers and phases are used interchangeably to mean layers which may interact at their interfaces. The order of casting may also be reversed, although it is preferred to cast the thin film first. The films may be allowed to dry under ambient conditions, or a heating device may be utilized. The first film need not be absolutely dry when the second film is cast on it; i.e., the first film may be tacky to the touch. It is believed that a skin forms on the top surface of the thick film after drying.

The solution of the cellulose acetate for the formation of the thin, more dense membrane component is formed by dissolving a cellulose acetate polymer in an inert organic solvent such as a ketone. Typical ketones are acetone, cyclohexanone, methylethylketone and the like. Mixtures of miscible solvents may also be used.

Concentration of the polymer in solvent may vary, as from 1 to 5% preferably 2 to 3%. The film is cast with any suitable film applicator such as will produce a final product film thickness of about 1—10 microns, preferably about 2—5 microns in thickness.

Typical electrochemical sensors which can be employed with the membrane of this invention include the Biostator glucose electrode of Miles Laboratories, Inc. See U.S. Patent No. 4,092,233.

The overall thickness of the membrane of the invention can vary from about 40 to about 100 microns, but is preferably approximately 70 microns.

The following drawings illustrate the invention in further detail and the invention will be more fully understood by reference to these drawings wherein:

Figure 1 is a vertical section view (partial) of a' polarographic cell utilizing the membrane of the present invention, and

Figure 2 is an enlarged view of a cross-section of the membrane of the present invention.

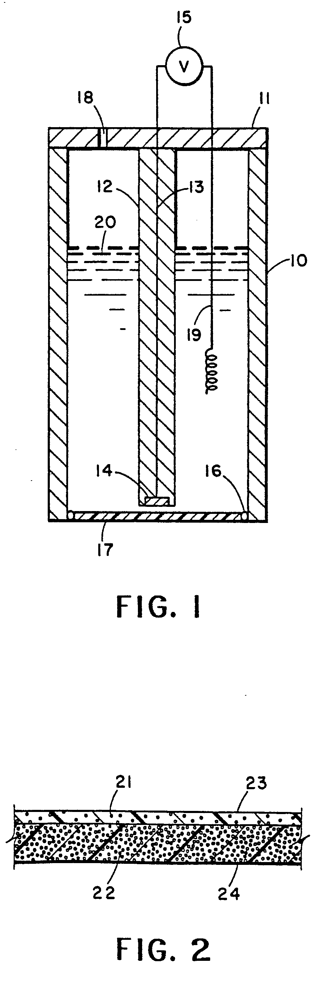

Referring to Figure 1, there is shown a polarographic cell assembly which includes a receptacle in the form of an electrically insulating container 10 made of a plastic or glass material or any other suitable material and which may be of any cross-sectional area and shape, but is preferably cylindrical. This is covered by an electrically insulating cap 11. Positioned within the receptacle is an electrically insulating member rod, or cylindrical column 12, which contains in it an electrical conductor 13. This conductor is connected to an active or exposed element 14 which may be platinum, gold, silver, graphite or the like.

A lead is attached to the electrode which passes through the rod or column and through the cap to be connected with a D.C. voltage source 15.

The lower end of the receptacle is provided with a support means 16 such as a ring or retainer and the membrane 17 in accordance with the present invention is supported over the end of the supporting receptacle nearest the central electrode and spaced a capillary distance from the active face of the electrode. The membrane can be held in position with any suitable means, for example, by an O-ring fitting into a circular groove or other convenient means in the receptacle. A current measuring instrument (not shown) is connected in series with the cell.

Typically, the receptacle is provided with a vent 18 to permit gases to escape if pressure inside the receptacle rises to a sufficiently high degree.

An annular space is provided between the central rod and the receptacle walls and receives a reference electrode 19 which may be, for example, silver chloride coated silver wire. The space 20 inbetween is at least partially and preferably completely filled with a liquid mixture of electrolyte which provides electrical contact between both electrodes and which may be introduced into the chamber through an aperture.

In polarographic measurements, two electrodes are commonly used, one of which is polarized and does not allow current to flow until depolarized by the substance being measured. In the cell structure shown in Figure 1, electrode 19 is the cathode and is polarized and frequently referred to as the reference electrode. The other electrode, electrode 14 as shown in Figure 1, functions as an anode and is not polarized in the presence of the substance being measured and therefore will not restrict the flow of relatively large current and is frequently referred to as the sensor electrode. Electrodes are shown in Figure 1, and are in an electrically insulating relation. The electrolyte material which occupies the chamber provides an electrical path between the two electrodes. Typical electrolytes include sodium or potassium chloride, buffers including carbonates, phosphates, bicarbonates, acetates, alkali or rare earth salts or other organic buffers or mixtures thereof can be used. The solvent for such an electrolyte can be water, glycols, glycerine and mixtures thereof as is well known in the art.

Figure 2 shows a composite membrane in cross-sectional detail. The nonhomogeneous composite membrane has a thin, dense membrane or layer 21 and a thick, less dense or porous membrane or layer 22 which membranes together form the composite membrane structure. The encapsulated enzyme shown symbolically by small circles is dispersed uniformly in the thick membrane or layer of the composite membrane. However, some of the encapsulated enzyme may diffuse into the thin membrane or layer during preparation of the membrane before the solvent for the cellulose acetate has evaporated. Membrane surface 24 is in electrical contact with the electrode. The composite membrane comprises the non-homogeneous combination of the two phases and the enzyme, the outer free surface 23 of which represents the test surface which is to be brought into contact with the solution to be analyzed.

In the preferred embodiment, the outer layer 24 which is in contact with the electrode is about 65 microns in thickness and the opposite layer in contact with the sample to be analyzed is about 2 microns. The overall thickness of the membrane is preferably about 70 microns.

The membrane of the present invention is a self-supporting film of a total thickness which may range from about 40 to 100 microns, preferably about 70 microns. The membrane may be shaped to any particular configuration or size or may be cut or dimensioned in any particular way to fit receptacles for polarographic cells or electrodes of any suitable dimension. It may, in particular, be fastened to an O-ring for use in an electrode such as described in U.S. Patent No. 4,092,233.

To fasten the membrane to a rubbery O-ring of an appropriate size, a gluing operation may be employed. The membrane may also be cast directly onto an electrode surface.

In addition to cellulose acetate, other polymers capable of being dissolved in solvents and undergoing phase inversion with the addition of a weak solvent or nonsolvent would be potential membrane materials. Such polymers include cellulose nitrate, ethylcellulose and other cellulose derivatives. In addition, polycarbonate is a suitable alternative if methylene chloride is employed as a solvent instead of acetone or other ketones.

As a substitute or alternative for the lower alcohols present in the phase inversion mixture formamide can be used.

Further variations and modifications of the invention will be apparent to those skilled in the art and are limited only by the scope of the claim.

## Claims

1. A membrane formed of polymer for use in a polarographic cell for the polarographic analysis of an unknown characterized in that it has uniformly distributed throughout an enzyme in the form of pockets of enzyme.

2. A membrane according to Claim 1, characterized in that said enzyme is mixed with a gel forming material and said pockets are globules of enzyme encapsulated in cellulose acetate.

3. A method of making a polymeric membrane for use with a polarographic cell utilizing immiscible polar and nonpolar solvents characterized in that it comprises the steps of forming a solution of an enzyme in a solvent and thereafter distributing said solution throughout a solution of a membrane material in a nonpolar solvent, thereafter casting a film of said membrane material and permitting the nonpolar solvent to evaporate, thereby distributing the enzyme throughout the membrane in pockets associated with the polar solvent.

4. A method according to Claim 3, characterized in that the polar solvent containing enzyme is dispersed in the nonpolar solvent containing membrane material by the use of agitation or ultrasonic mixing.

5. A method according to Claim 4, characterized in that the enzyme and the polar solvent form a mixture capable of forming a gel after the membrane has been cast so that the polar solvent and the enzyme remain dispersed in globules throughout the membrane material.

6. A method according to Claims 3 to 5, characterized in that a second membrane is formed adjacent to and in contact with the membrane containing said enzyme.

7. A method according to Claims 3 to 6, characterized in that the enzyme is glucose oxidase.

## Patentansprüche

1. Aus einem Polymer für die Verwendung in einer polarografischen Zelle für die polarografische Analyse von etwas Unbekanntem gebildete Membran, dadurch gekennzeichnet, dass diese überall in gleichmässiger Verteilung ein Enzym in Form von Enzymtaschen enthält.

2. Membran gemäss Anspruch 1, dadurch gekennzeichnet, dass das Enzym mit einem gelbildenden Material vermischt ist und die Taschen in Celluloseacetat eingekapselte Enzymkügelchen sind.

3. Verfahren zur Herstellung einer Polymermembran für die Verwendung mit einer polarografischen Zelle unter Verwendung von unmischbaren polaren und nicht-polaren Lösungsmitteln, dadurch gekennzeichnet, dass es die Stufen umfasst: Ausbildung einer Enzymlösung in einem Lösungsmittel, anschliessend vollständige Verteilung der Lösung in einer Lösung aus einem Membranmaterial in einem nicht-polaren Lösungsmittel, anschliessend Vergiessen eines Films aus dem Membranmaterial, wobei man das nicht-polare Lösungsmittel verdampfen lässt und wodurch das Enzym vollständig in der Membran in Taschen, zusammen mit dem polaren Lösungsmittel, verteilt wird.

4. Verfahren gemäss Anspruch 3, dadurch gekennzeichnet, dass das Enzym enthaltende, polare Lösungsmittel in dem Membranmaterial enthaltenden, nicht-polaren Lösungsmittel durch Rühren oder Ultraschallmischen dispergiert ist.

5. Verfahren gemäss Anspruch 4, dadurch gekennzeichnet, dass das Enzym und das polare Lösungsmittel eine Mischung bilden, die in der Lage ist, einen Gel zu bilden, nachdem die Membran vergossen wurde, so dass das polare Lösungsmittel und das Enzym in Kügelchen verteilt in dem ganzen Membranmaterial dispergiert bleibt.

6. Verfahren gemäss Ansprüchen 3 bis 5, dadurch gekennzeichnet, dass man eine zweite Membran anliegend an und in Kontakt mit der Enzym enthaltenden Membran ausbildet.

7. Verfahren gemäss Ansprüchen 3 bis 6, dadurch gekennzeichnet, dass das Enzym Glucoseoxidase ist.

**Revendications**

1. Membrane formée d'un polymère pour l'utilisation dans une cellule polarographique pour l'analyse polarographique d'une substance inconnue, caractérisée en ce qu'elle renferme une enzyme uniformément distribuée sous la forme de poches d'enzyme.

2. Membrane selon la revendication 1, caractérisée en ce que ladite enzyme est mélangée avec une matière formant un gel et lesdites poches sont des globules de l'enzyme encapsulée dans l'acétate de cellulose.

3. Procédé de fabrication d'une membrane polymère pour l'utilisation avec une cellule polarographique utilisant des solvants polaires et non polaires non miscibles, caractérisé en ce qu'il comprend les étapes de formation d'une solution d'une enzyme dans un solvant et ensuite de distribution de ladite solution au sein d'une solution d'une matière de membrane dans un solvant non polaire, puis de coulée d'un film de ladite matière de membrane et d'évaporation des solvants non polaires, de manière à distribuer l'enzyme au sein de la membrane dans des poches associées avec le solvant polaire.

4. Procédé selon la revendication 3, caractérisé en ce que le solvant polaire contenant l'enzyme est dispersé dans le solvant non polaire contenant la matière de membrane par utilisation d'agitation ou de mélange par ultrasons.

5. Procédé selon la revendication 4, caractérisé en ce que l'enzyme et le solvant polaire forment un mélange capable de former un gel après que la membrane a été coulée, de sorte que le solvant polaire et l'enzyme restent dispersés dans des globules au sein de la matière de membrane.

6. Procédé selon les revendications 3 à 5, caractérisé en ce qu'une seconde membrane est formée au voisinage et en contact de la membrane contenant ladite enzyme.

7. Procédé selon les revendications 3 à 6, caractérisé en ce que l'enzyme est la glucose-oxydase.

FIG. 1

FIG. 2